# EUROPEAN PATENT SPECIFICATION

(11) **EP 4 504 718 B1**
(45) Date of publication and mention of the grant of the patent: **10.06.2026**
(21) Application number: 23718993.1
(22) Date of filing: 06.04.2023
(51) Int. Cl.: C07D 403/04, A61K 31/506, A61P 35/00

(54) **IMPROVED PROCESS FOR THE MANUFACTURE OF OSIMERTINIB**
VERBESSERTES VERFAHREN ZUR HERSTELLUNG VON OSIMERTINIB
PROCÉDÉ AMÉLIORÉ DE PRODUCTION D'OSIMERTINIB

(30) Priority: 07.04.2022 US 202263362622 P
(43) Date of publication of application: 12.02.2025
(73) Proprietor: Astrazeneca AB, 151 85 Södertälje (SE)
(72) Inventor: MULLEN, Alexander Kieron, Cambridge Cambridgeshire CB2 0AA (GB); POZZOLI, Alessandro, Cambridge Cambridgeshire CB2 0AA (GB)
(74) Representative: AstraZeneca Intellectual Property
(86) International application number: PCT/EP2023/059132
(87) International publication number: WO 2023/194531

(56) References cited:
- CN-A- 106 883 216
- CN-A- 109 134 435
- IN-A- 201741 031 902

## Description

This specification relates an improved process for the manufacture of osimertinib.

### Introduction

Osimertinib (AZD9291) is a third generation EGFR Tyrosine Kinase Inhibitor (TKI). Osimertinib is disclosed in WO 2013/014448. Osimertinib has the following chemical structure:

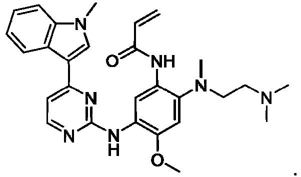

Osimertinib mesylate is an approved treatment for non-small cell lung cancer (NSCLC), and is also known as TAGRISSO^{™}.

WO 2013/014448 discloses the following synthesis of osimertinib.

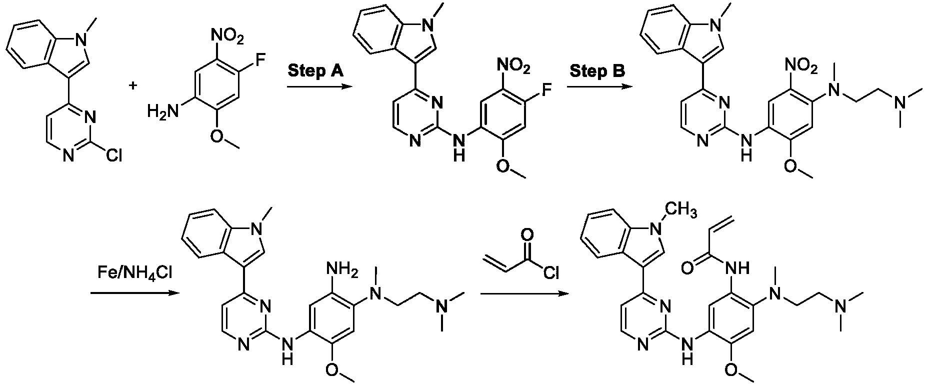

This synthetic process includes the step of making a compound of Formula (I) (where R¹ = CH₃) from a compound of Formula (II) and a compound of Formula (III) (Step A herein). WO 2013/014448 discloses the use of p-toluenesulfonic acid and 2-pentanol at 85 °C for 3 hours for this step. CN109134435 discloses acetonitrile (MeCN) as an alternative solvent for this step, with the reaction heated to 85 °C for 12 hours.

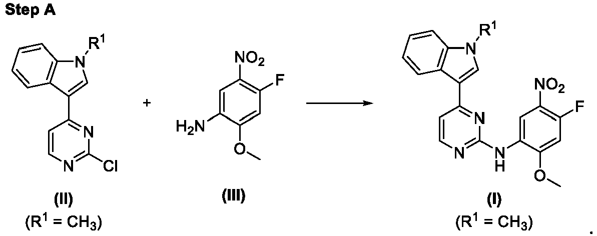

After Step A, WO 2013/014448 discloses that the compound of Formula (I) (where R¹ = CH₃) was isolated and dried under vacuum. WO 2013/014448 discloses that the compound of Formula (I) was then converted to a compound of Formula (IV) by reaction with a compound of Formula (V) (*N,N',N'-*trimethyl-ethane-1,2-diamine) (Step B herein) in the presence of N,N-diisopropylethylamine (DIPEA) and 2,2,2-trifluoroethanol at 140°C for 1 h.

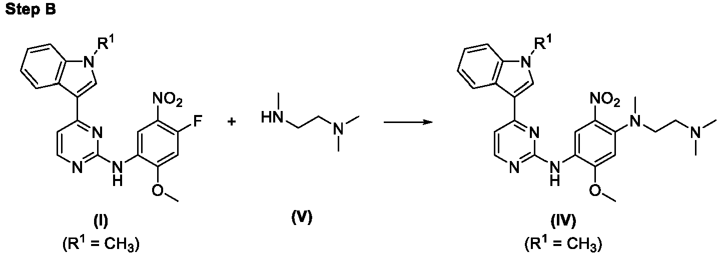

Although the synthetic route disclosed in the WO 2013/014448 provides a reliable method for producing osimertinib, it is desirable to improve the economy of the process for large-scale manufacture to minimise environmental impact and reduce cost of goods.

To this end, a new telescoped process was developed to combine Step A and Step B.

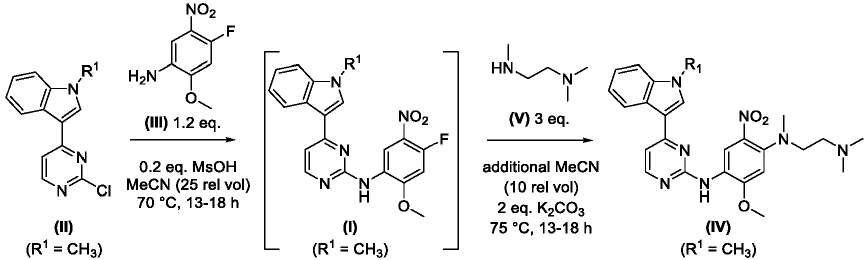

This new telescoped process avoided the need to isolate the compound of Formula (I), improving the overall economy of the manufacture of EGFR TKls, such as osimertinib. However, one drawback of this process was that a high relative volume of acetonitrile (MeCN) was required. As set out above, the first reaction to form a compound of Formula (I) required 25 relative volumes of acetonitrile, with a further 10 relative volumes of acetonitrile added for the second reaction to form a compound of Formula (IV). Upon completion of the second reaction, the reaction mixture was diluted with a further 15 relative volumes of acetonitrile and purified by a hot filtration to remove inorganics, washing with a further 2 relative volumes of acetonitrile. As such, a total of 52 relative volumes of acetonitrile was required. If less acetonitrile was used, it was observed that the compound of Formula (IV) crystallised prior to the hot filtration. This uncontrolled crystallisation of the compound of Formula (IV) was detrimental to the purity of the isolated material. Furthermore, this uncontrolled crystallisation made it more difficult to isolate the compound of Formula (IV) from any inorganic solid present at the end of the reaction by filtration.

There is therefore a need for a further improved process for the synthesis of the compound of Formula (IV), as part of the manufacture of EGFR TKls, such as osimertinib.

### General Description

According to one aspect of the specification there is provided a process for the production of a compound of Formula (I): 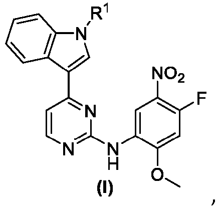
or a salt thereof, comprising a reaction of a compound of Formula (II): 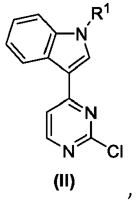
or a salt thereof, and a compound of Formula (III): 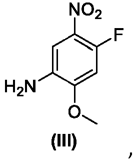
or a salt thereof, wherein the reaction is performed in the presence of an acid and benzonitrile,
wherein R¹ is C₁₋₃ alkyl or cyclopropyl.

In another aspect of the specification there is provided a process for the production of a compound of Formula (IV) 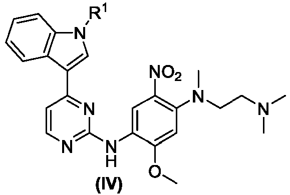
or a salt thereof, comprising the following steps
   (i) the production of a compound of Formula (I), or a salt thereof, comprising a reaction of a compound of Formula (II), or a salt thereof, and a compound of Formula (III), or a salt thereof, in the presence of an acid and benzonitrile; and
   (ii) the production of the compound of Formula (IV), of a salt thereof, comprising the reaction of the compound of Formula (I), or a salt thereof, and a compound of Formula (V) 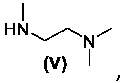
or a salt thereof, in the presence of benzonitrile,
wherein R¹ is C₁₋₃ alkyl or cyclopropyl.

Surprisingly, the use of benzonitrile as a solvent for the production of a compound of Formula (I) yields a crude reaction mixture that is suitable for the production of a compound of Formula (IV) without a need to isolate the compound of Formula (I). This telescoped sequence of Step A and Step B reduces the environmental impact and improves the overall cost of goods for the manufacture of EGFR TKls, such as osimertinib.

Furthermore, by using benzonitrile as solvent it is possible to telescope this sequence using fewer relative volumes of solvent compared to acetonitrile. This is because when benzonitrile was used, the compound of Formula (IV) remained in solution at significantly higher concentrations that was possible using acetonitrile.

In embodiments, the reaction of the compound of Formula (I), or a salt thereof, and a compound of Formula (V), or a salt thereof, is performed in the presence of DBU. This has the further advantage of avoiding the need for a hot filtration to remove solid impurities at the end of the reaction to form the compound of Formula (IV). Consequently, it is possible to avoid the need for specialised hot filtration equipment and reduce manufacturing time.

As used herein, the term **"molar equivalents"** (or "eq.") refers to molar equivalents with respect to the compound of Formula (II), or a salt thereof.

As used herein, the term **"relative volume"** (or "rel vol") means the volume of solvent in litres required relative to the charge of the compound of Formula (II), or a salt thereof, in kilograms.

As used herein, the term "MsOH" refers to methanesulfonic acid and **"MeCN"** refers to acetonitrile.

As used herein, there term "C₁₋₃ alkyl" refers to both straight and branched chain saturated hydrocarbon radicals having 1, 2 or 3 carbon atoms. Examples of C₁₋₃ alkyl are methyl, ethyl, n-propyl and i-propyl.

As used herein the term **"telescope"** refers to the process of performing two reactions sequentially without the isolation of the product of the first reaction. Herein, square brackets are used to indicate that a material is not isolated before being subjected to the next reaction in the sequence.

Units, prefixes, and symbols are denoted in their International System of Units (SI) accepted form. Numeric ranges are inclusive of the numbers defining the range.

### Detailed Description

As noted above, this specification provides a process for the production of a compound of Formula (I): 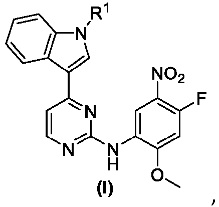
or a salt thereof, comprising a reaction of a compound of Formula (II): 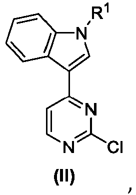
or a salt thereof, and a compound of Formula (III): 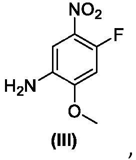
or a salt thereof, wherein the reaction is performed in the presence of an acid and benzonitrile wherein R¹ is C₁₋₃ alkyl or cyclopropyl.

Where R¹ is methyl, the free base of the compound of Formula (I) is known by the chemical name N-(4-fluoro-2-methoxy-5-nitrophenyl)-4-(1-methyl-1*H*-indol-3-yl)-2-pyrimidinamine. In embodiments, the compound of Formula (I) is N-(4-fluoro-2-methoxy-5-nitrophenyl)-4-(1-methyl-1H-indol-3-yl)-2-pyrimidinamine.

Where R¹ is methyl, the free base of the compound of Formula (II) is known by the chemical name 3-(2-chloro-4-pyrimidinyl)-1-methyl-1H-indole (AZD9291 Chloropyrimidine). In embodiments, the compound of Formula (II) is 3-(2-chloro-4-pyrimidinyl)-1-methyl-1*H*-indole. The compound of Formula (II) may also be known by the name 3-(2-chloropyrimidin-4-yl)-1-methyl-1H-indole.

The free base of the compound of Formula (III) is known by the chemical name 4-fluoro-2-methoxy-5-nitroaniline (AZD9291 Nitroaniline). In embodiments, the compound of Formula (III) is 4-fluoro-2-methoxy-5-nitroaniline.

Suitable acids are Bronsted acids, for example, carboxylic acids, sulfonic acids and mineral acids.

In embodiments, the acid is selected from a sulfonic acid, a carboxylic acid, and a mineral acid.

In embodiments, the acid is a sulfonic acid. In further embodiments, the sulfonic acid is selected from methanesulfonic acid, benzenesulfonic acid and p-toluenesulfonic acid.

In embodiments, the acid is methanesulfonic acid.

In embodiments, the acid is a carboxylic acid. In further embodiments, the carboxylic acid is selected from (C₁₋₇hydrocarbyl)COOH, formic acid, trichloroacetic acid and trifluoroacetic acid. An example of a (C₃hydrocarbyl)-COOH is n-butanoic acid. An example of a (C₆hydrocarbyl)COOH is benzoic acid. In further embodiments, the carboxylic acid is selected from acetic acid and trifluoroacetic acid.

In embodiments, the acid is a mineral acid. In further embodiments, the mineral acid is selected from hydrochloric acid, sulfuric acid and phosphoric acid.

In embodiments, at least 0.02 molar equivalents of acid is used. In further embodiments, 0.02-1 molar equivalents of acid is used. In further embodiments, 0.02-0.30 molar equivalents of acid is used. In further embodiments, 0.04 to 0.30 molar equivalents of acid is used. In further embodiments, 0.02 to 0.15 molar equivalents of acid is used. In further embodiments, 0.04 to 0.15 molar equivalents of acid is used. In further embodiments, 0.06 to 0.15 molar equivalents of acid is used. In further embodiments, 0.04 to 0.12 molar equivalents of acid is used. In further embodiments, 0.06 to 0.12 molar equivalents of acid is used. In further embodiments, about 0.1 molar equivalents of acid is used. In further embodiments, 0.1 molar equivalents of acid is used. In further embodiments, about 0.075 molar equivalents of acid is used. In further embodiments, 0.075 molar equivalents of acid is used. It is to be understood that the amount (molar equivalents) of acid is relative to the amount of the compound of Formula (II), or a salt thereof.

In embodiments, the reaction of a compound of Formula (II), or a salt thereof, and a compound of Formula (III), or a salt thereof, is performed at a temperature of at least 60 °C. In further embodiments, the reaction of a compound of Formula (II), or a salt thereof, and a compound of Formula (III), or a salt thereof, is performed at a temperature in the range 60 to 130 °C. In further embodiments, the reaction is performed at a temperature in the range 80 to 130 °C. In further embodiments, the reaction is performed at a temperature in the range 60 to 120 °C. In further embodiments, the reaction is performed at a temperature in the range 80 to 120 °C. In further embodiments, the reaction is performed at a temperature in the range 90 to 110 °C. In further embodiments, the reaction is performed at a temperature in the range 100 to 110 °C. In further embodiments, the reaction is performed at a temperature of about 100 °C. In further embodiments, the reaction is performed at a temperature of 100 °C. In further embodiments, the reaction is performed at a temperature of about 105 °C. In further embodiments, the reaction is performed at a temperature of 105 °C.

In embodiments, the reaction of a compound of Formula (II), or a salt thereof, and a compound of Formula (III), or a salt thereof, is performed at a temperature in the range 60 to 130 °C for up to 24 hours. In further embodiments, the reaction is performed at a temperature in the range 80 to 120 °C for 3 to 5 hours. In further embodiments, the reaction is performed at a temperature in the range 90 to 110 °C for 3 to 5 hours.

In embodiments, the reaction of a compound of Formula (II), or a salt thereof, and a compound of Formula (III), or a salt thereof, is performed with at least 4 relative volumes of benzonitrile. In further embodiments, the reaction is performed with 4 to 10 relative volumes of benzonitrile. In further embodiments, the reaction is performed with 4 to 6 relative volumes of benzonitrile. In further embodiments, the reaction is performed with about 5 relative volumes of benzonitrile. In further embodiments, the reaction is performed with 5 relative volumes of benzonitrile.

In embodiments, the reaction of a compound of Formula (II), or a salt thereof, and a compound of Formula (III), or a salt thereof, is performed with at least 1 L of benzonitrile/Mole of the compound of Formula (II), or a salt thereof. In further embodiments, the reaction is performed with 1 to 2.5 L of benzonitrile/Mole of the compound of Formula (II), or a salt thereof. In further embodiments, the reaction is performed with 1 to 1.5 L of benzonitrile/Mole of the compound of Formula (II), or a salt thereof. In further embodiments, the reaction is performed with about 1.2 L of benzonitrile/Mole of the compound of Formula (II), or a salt thereof. In further embodiments, the reaction is performed with 1.2 L of benzonitrile/Mole of the compound of Formula (II), or a salt thereof.

In embodiments, the reaction of a compound of Formula (II), or a salt thereof, and a compound of Formula (III), or a salt thereof, is performed with at least 50 mMoles of the compound of Formula (II), or a salt thereof. In further embodiments, the reaction is performed with at least 80 mMoles of the compound of Formula (II), or a salt thereof.

In embodiments, the reaction of a compound of Formula (II), or a salt thereof, and a compound of Formula (III), or a salt thereof, is performed with at least 1.0 molar equivalents of the compound of Formula (III), or a salt thereof. In further embodiments, the reaction is performed with 1.0-1.5 molar equivalents of the compound of Formula (III), or a salt thereof. In further embodiments, the reaction is performed with 1.0-1.3 molar equivalents of the compound of Formula (III), or a salt thereof. In further embodiments, the reaction is performed with at 1.0-1.2 molar equivalents of the compound of Formula (III), or a salt thereof. In further embodiments, the reaction is performed with 1.05-1.2 molar equivalents of the compound of Formula (III), or a salt thereof. In further embodiments, the reaction is performed with 1.05-1.15 molar equivalents of the compound of Formula (III), or a salt thereof. In further embodiments, the reaction is performed with about 1.1 molar equivalents of the compound of Formula (III), or a salt thereof. In further embodiments, the reaction is performed with 1.1 molar equivalents of the compound of Formula (III), or a salt thereof. It is to be understood that the amount (molar equivalents) of compound of Formula (III), or a salt thereof, is relative to the amount of the compound of Formula (II), or a salt thereof.

In embodiments, there is provided the reaction of a compound of Formula (II), or a salt thereof, and a compound of Formula (III), or a salt thereof, wherein R¹ is C₁₋₃ alkyl, such as methyl.

In another aspect of the specification there is provided a process for the production of a compound of Formula (IV) 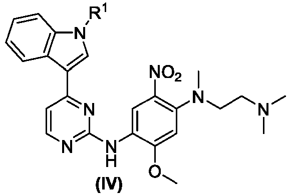
or a salt thereof, comprising the following steps
   (i) the production of a compound of Formula (I), or a salt thereof, comprising a reaction of a compound of Formula (II), or a salt thereof, and a compound of Formula (III), or a salt thereof, in the presence of an acid and benzonitrile; and
   (ii) the production of the compound of Formula (IV), of a salt thereof, comprising the reaction of the compound of Formula (I), or a salt thereof, and a compound of Formula (V) 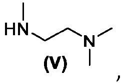
or a salt thereof, in the presence of benzonitrile,
wherein R¹ is C₁₋₃ alkyl or cyclopropyl.

In embodiments, step (i) is as described in any of the aforementioned embodiments.

Where R¹ is methyl, the free base of the compound of Formula (IV) is known by the chemical name *N*-[2-(dimethylamino)ethyl]-5-methoxy-*N*-methyl-*N*'-[4-(1-methyl-1H-indol-3-yl)-2-pyrimidinyl]-2-nitro-1,4-benzenediamine (AZD9291 Nitrodiamine). In embodiments, the compound of Formula (IV) is *N*-[2-(dimethylamino)ethyl]-5-methoxy-*N*-methyl-*N*'-[4-(1-methyl-1H-indol-3-yl)-2-pyrimidinyl]-2-nitro-1,4-benzenediamine. Where R¹ is methyl, the compound of Formula (IV) may also be known by the name N¹-(2-(dimethylamino)ethyl)-5-methoxy-N¹-methyl-N⁴-(4-(1-methyl-1H-indol-3-yl)pyrimidin-2-yl)-2-nitrobenzene-1,4-diamine.

The free base of the compound of Formula (V) is known by the chemical name *N,N,N'-*trimethylethylenediamine (TriMEDA). In embodiments, the compound of Formula (V) is *N,N,N'-*trimethylethylenediamine. The compound of Formula (V) may also be known by the name N¹,N¹,N²-trimethylethane-1,2-diamine.

In embodiments, the reaction of the compound of Formula (I), or a salt thereof, and a compound of Formula (V), or a salt thereof, is performed in the presence of a base. Suitable bases are Bronsted bases, for example, an organic base or an inorganic base.

In embodiments, the base is an amidine base or a guanidine base.

In embodiments, the base is 1,8-diazabicyclo(5.4.0)undec-7-ene (DBU), 1,1,3,3-tetramethylguanidine (TMG), 1,5-diazabicyclo[4.3.0]non-5-ene (DBN), 7-methyl-1,5,7-triazabicyclo(4.4.0)dec-5-ene (MTBD) or triazabicyclodecene (TBD). In further embodiments, the base is DBU.

In embodiments, the base is selected from potassium carbonate (K₂CO₃), potassium hydrogen carbonate (KHCO₃), sodium carbonate (Na₂CO₃), sodium hydrogen carbonate (NaHCO₃), sodium hydroxide (NaOH), potassium hydroxide (KOH), lithium hydroxide (LiOH), caesium hydroxide (CsOH), calcium hydroxide (Ca(OH)₂), calcium carbonate (CaCO₃), barium hydroxide (Ba(OH)₂) and caesium carbonate (Cs₂CO₃).

In embodiments, the reaction of the compound of Formula (I), or a salt thereof, and a compound of Formula (V), or a salt thereof, is performed with at least 2 molar equivalents of the base. In further embodiments, the reaction is performed with at least 2.2 molar equivalents of the base. In further embodiments, the reaction is performed with 2.0 to 2.5 molar equivalents of the base. In further embodiments, the reaction is performed with 2.0 to 2.4 molar equivalents of the base. In further embodiments, the reaction is performed with 2.2 to 2.5 molar equivalents of the base. In further embodiments, the reaction is performed with 2.2 to 2.4 molar equivalents of the base. In further embodiments, the reaction is performed with about 2.3 molar equivalents of the base. In further embodiments, the reaction is performed with 2.3 molar equivalents of the base. It is to be understood that the amount (molar equivalents) of base is relative to the amount of the compound of Formula (II), or a salt thereof.

In embodiments, the reaction of the compound of Formula (I), or a salt thereof, and a compound of Formula (V), or a salt thereof, is performed in the presence of a fluoride scavenger. In further embodiments, the fluoride scavenger is a calcium salt. In further embodiments, the fluoride scavenger is selected from calcium hydroxide (Ca(OH)₂), calcium carbonate (CaCO₃), calcium propionate (Ca(C₂H₅COO)₂), calcium acetate ((Ca(OAc)₂), calcium citrate, calcium gluconate and calcium chloride (CaCl₂).

In embodiments, the reaction of the compound of Formula (I), or a salt thereof, and a compound of Formula (V), or a salt thereof, is performed with at least 2 molar equivalents of the fluoride scavenger. In further embodiments, the reaction is performed with at least 2.2 molar equivalents of the fluoride scavenger. In further embodiments, the reaction is performed with 2.0 to 2.5 molar equivalents of the fluoride scavenger. In further embodiments, the reaction is performed with 2.0 to 2.4 molar equivalents of the fluoride scavenger. In further embodiments, the reaction is performed with 2.2 to 2.5 molar equivalents of the fluoride scavenger. In further embodiments, the reaction is performed with 2.2 to 2.4 molar equivalents of the fluoride scavenger. In further embodiments, the reaction is performed with about 2.3 molar equivalents of the fluoride scavenger. In further embodiments, the reaction is performed with 2.3 molar equivalents of the fluoride scavenger. It is to be understood that the amount (molar equivalents) of fluoride scavenger is relative to the amount of the compound of Formula (II), or a salt thereof.

In embodiments, the reaction of the compound of Formula (I), or a salt thereof, and a compound of Formula (V), or a salt thereof, is performed with at least 1 molar equivalent of the compound of Formula (V), or a salt thereof. In embodiment, the reaction is performed with at least 1.3 molar equivalents of the compound of Formula (V), or a salt thereof. In embodiment, the reaction is performed with 1.3 to 3 molar equivalents of the compound of Formula (V), or a salt thereof. In embodiment, the reaction is performed with 1.5 to 2.5 molar equivalents of the compound of Formula (V), or a salt thereof. In embodiment, the reaction is performed with 1.5 to 2.2 molar equivalents of the compound of Formula (V), or a salt thereof. In embodiment, the reaction is performed with 1.8 to 2.5 molar equivalents of the compound of Formula (V), or a salt thereof. In embodiment, the reaction is performed with 1.8 to 2.2 molar equivalents of the compound of Formula (V), or a salt thereof In embodiment, the reaction is performed with about 2 molar equivalents of the compound of Formula (V), or a salt thereof. In embodiment, the reaction is performed with 2 molar equivalents of the compound of Formula (V), or a salt thereof. It is to be understood that the amount (molar equivalents) of compound of Formula (V), or a salt thereof, is relative to the amount of the compound of Formula (II), or a salt thereof.

In embodiments, the reaction of the compound of Formula (I), or a salt thereof, and a compound of Formula (V), or a salt thereof, is performed at a temperature of at least 40 °C, such as at least 60 °C. In further embodiments, the reaction is performed at a temperature in the range 40 to 100 °C. In further embodiments, the reaction is performed at a temperature in the range 60 to 100 °C. In further embodiments, the reaction is performed at a temperature in the range 60 to 90 °C. In further embodiments, the reaction is performed at a temperature in the range 70 to 100 °C. In further embodiments, the reaction is performed at a temperature in the range 70 to 90 °C. In further embodiments, the reaction is performed at a temperature in the range 70 to 85 °C. In further embodiments, the reaction is performed at a temperature of about 70 °C. In further embodiments, the reaction is performed at a temperature of about 70 °C. In further embodiments, the reaction is performed at a temperature of about 80 °C. In further embodiments, the reaction is performed at a temperature of about 80 °C.

In embodiments, there is provided the reaction of the compound of Formula (I), or a salt thereof, and a compound of Formula (V), or a salt thereof, wherein R¹ is C₁₋₃ alkyl, such as methyl.

In embodiments, step (i) and step (ii) are performed sequentially without the isolation of the compound of Formula (I), or a salt thereof, from the benzonitrile of step (i). In further embodiments, step (ii) is performed without the addition of further benzonitrile.

In embodiments, step (i) and step (ii) are telescoped.

### Examples

### Example 1: Telescoped Synthesis of N-[2-(dimethylamino)ethyl]-5-methoxy-N-methyl-N'-[4-(1-methyl-1H-indol-3-yl)-2-pyrimidinyl]-2-nitro-1,4-benzenediamine (AZD9291 Nitrodiamine)

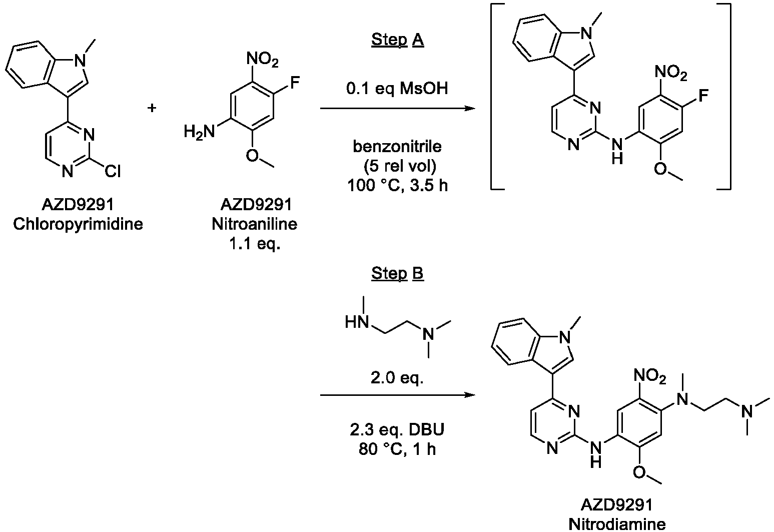

### Step A

An agitated mixture of 3-(2-chloro-4-pyrimidinyl)-1-methyl-1H-indole (AZD9291 Chloropyrimidine, 25.00 g, 1.00 mol eq.) and 4-fluoro-2-methoxy-5-nitroaniline (AZD9291 Nitroaniline, 21.00 g, 1.10 mol eq.) in benzonitrile (125 mL, 5.0 rel vol) at 60°C was charged with methane sulfonic acid (0.99 g, 0.1 mol eq.). The resulting mixture was heated to 100 °C for 3.5-4 hours. The reaction mixture was then cooled to 40°C.

### Step B

The reaction mixture was then charged with 1,8-diazabicyclo[5.4.0]undec-7-ene (DBU, 37.8 g, 37.1 ml, 2.30 mol eq.), maintaining the temperature below 45°C. The reaction mixture was then charged with N,N,N'-trimethylethylenediamine (TriMEDA, 21.0 g, 26.3 mL, 2.0 mol eq.), maintaining the temperature below 45 °C. The reaction mixture was then heated to 80 °C for 1 hour, and then cooled to 70 °C and charged with AZD9291 Nitrodiamine seed. The mixture was then held at 70 °C for 1 hour, then cooled to 5 °C at a rate of 0.1 °C/min over 11 hours. Solid material was isolated by vacuum filtration, washing twice with isopropanol (125 mL, then 75 mL). The solid material was dried under vacuum at 50 °C to give the title compound (42.1 g, 86.3%); ¹H NMR (500 MHz, CDCI3, 27°C): 9.55, 8.38, 8.37, 8.26, 8.16, 8.15, 7.52, 7.40, 7.38, 7.32, 7.31, 7.30, 7.30, 7.29, 7.27, 7.26, 7.26, 7.18, 7.17, 6.66, 3.97, 3.93, 3.29, 3.28, 3.26, 2.90, 2.58, 2.57, 2.55, 2.26, 1.80. ¹³C NMR (126 MHz, CDCI3, 27°C): 161.50, 158.97, 157.61, 151.98, 142.66, 137.79, 133.66, 132.76, 125.48, 122.93, 122.06, 121.03, 120.41, 116.02, 113.40, 109.85, 107.90, 101.38, 56.78, 55.76, 53.90, 45.61, 40.93, 33.16. [M+H]⁺: 476.32.

AZD9291 Nitrodiamine seed may be prepared by the recrystallization of AZD9291 Nitrodiamine (accessible according to WO 2013/014448) in benzonitrile. For example, AZD9291 Nitrodiamine may be dissolved in a minimum of benzonitrile at 70 °C, then cooled to 5 °C at a rate of 0.1 °C/min over 11 hours.

The above description of illustrative embodiments is intended only to acquaint others skilled in the art with the Applicant's specification, its principles, and its practical application so that others skilled in the art may readily adapt and apply the specification in its numerous forms, as they may be best suited to the requirements of a particular use. This description and its specific examples, while indicating embodiments of this specification, are intended for purposes of illustration only. This specification, therefore, is not limited to the illustrative embodiments described in this specification, and may be variously modified. In addition, it is to be appreciated that various features of the specification that are, for clarity reasons, described in the context of separate embodiments, also may be combined to form a single embodiment. Conversely, various features of the specification that are, for brevity reasons, described in the context of a single embodiment, also may be combined to form sub-combinations thereof.

## Claims

1. A process for the production of a compound of Formula (I): 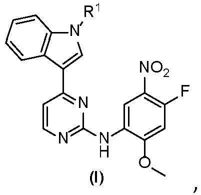
or a salt thereof, comprising a reaction of a compound of Formula (II): 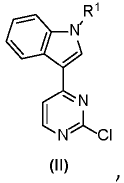
or a salt thereof, and a compound of Formula (III): 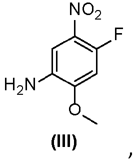
or a salt thereof, wherein the reaction is performed in the presence of an acid and benzonitrile,
wherein R¹ is C₁₋₃ alkyl or cyclopropyl.

2. A process as claimed in claim 1, wherein the reaction is performed with 0.02 to 0.3 molar equivalents of the acid, such as 0.05 to 0.15 molar equivalents of the acid.

3. A process as claimed in claim 1 or claim 2, wherein the acid is
(a) a sulfonic acid,
(b) methanesulfonic acid, benzenesulfonic acid or p-toluenesulfonic acid, or
(c) methanesulfonic acid.

4. A process as claimed in any one of claims 1 to 3, wherein the reaction is performed at a temperature in the range 60 to 130 °C, such as 90 to 110 °C.

5. A process as claimed in any one of claims 1 to 4, wherein the reaction is performed with 3 to 10 relative volumes of benzonitrile, such as 4 to 6 relative volumes of benzonitrile, wherein the term "relative volume" (or "rel vol") means the volume of solvent in litres required relative to the charge of the compound of Formula (II), or a salt thereof, in kilograms.

6. A process as claimed in any one of claims 1 to 5, wherein the reaction is performed with 1.0 to 1.5 molar equivalents of the compound of Formula (III), or a salt thereof.

7. A process for the production of a compound of Formula (IV) 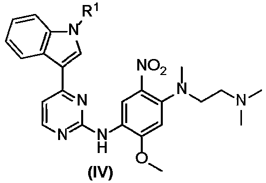
or a salt thereof, comprising the following steps
(i) the production of a compound of Formula (I), or a salt thereof, as claimed in any one of claims 1 to 6; and
(ii) the production of the compound of Formula (IV), of a salt thereof, comprising the reaction of the compound of Formula (I), or a salt thereof, and a compound of Formula (V) 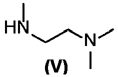
or a salt thereof, in the presence of benzonitrile,
wherein R¹ is C₁₋₃ alkyl or cyclopropyl.

8. A process as claimed in claim 7, wherein the reaction of the compound of Formula (I), or a salt thereof, and a compound of Formula (V), or a salt thereof, is performed in the presence of a base.

9. A process as claimed in claim 8, wherein the reaction of the compound of Formula (I), or a salt thereof, and a compound of Formula (V), or a salt thereof, is performed with 2.0 to 2.5 molar equivalents of the base.

10. A process as claimed in claim 8 or claim 9, wherein the base is
(a) 1,8-diazabicyclo(5.4.0)undec-7-ene (DBU), 1,1,3,3-tetramethylguanidine (TMG), 1,5-diazabicyclo[4.3.0]non-5-ene (DBN), 7-methyl-1,5,7-triazabicyclo(4.4.0)dec-5-ene (MTBD) or triazabicyclodecene (TBD), or
(b) DBU.

11. A process as claimed in any one of claims 7 to 10, wherein the reaction of the compound of Formula (I), or a salt thereof, and a compound of Formula (V), or a salt thereof, is performed in the presence of a fluoride scavenger.

12. A process as claimed in any one of claims 7 to 11, wherein the reaction of the compound of Formula (I), or a salt thereof, and a compound of Formula (V), or a salt thereof, is performed with 1.8 to 2.2 molar equivalents of the compound of Formula (V), or a salt thereof.

13. A process as claimed in any one of claims 7 to 12, wherein the reaction of the compound of Formula (I), or a salt thereof, and a compound of Formula (V), or a salt thereof, is performed at a temperature in the range 40 to 100 °C, such as 70 to 90 °C.

14. A process as claimed in any one of claims 7 to 13, wherein step (i) and step (ii) are performed sequentially without the isolation of the compound of Formula (I), or a salt thereof, from the benzonitrile of step (i).

15. A process as claimed in any one of claims 1 to 14, wherein R¹ is methyl.

## Patentansprüche

1. Verfahren für die Herstellung einer Verbindung von Formel (I): 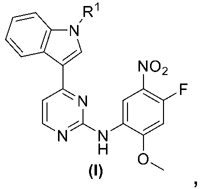
oder eines Salzes davon, umfassend ein Umsetzen einer Verbindung von Formel (II): 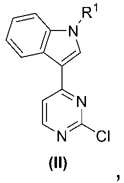
oder eines Salzes davon, und einer Verbindung von Formel (III): 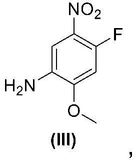
oder eines Salzes davon, wobei das Umsetzen in der Gegenwart einer Säure und Benzonitril durchgeführt wird,
wobei R¹ C₁₋₃-Alkyl oder Cyclopropyl ist.

2. Verfahren nach Anspruch 1, wobei das Umsetzen mit 0,02 bis 0,3 Moläquivalenten der Säure, wie 0,05 bis 0,15 Moläquivalenten der Säure, durchgeführt wird.

3. Verfahren nach Anspruch 1 oder 2, wobei die Säure
(a) eine Sulfonsäure,
(b) Methansulfonsäure, Benzolsulfonsäure oder p-Toluolsulfonsäure, oder
(c) Methansulfonsäure ist.

4. Verfahren nach einem der Ansprüche 1 bis 3, wobei das Umsetzen bei einer Temperatur in dem Bereich von 60 bis 130 °C, wie 90 bis 110 °C, durchgeführt wird.

5. Verfahren nach einem der Ansprüche 1 bis 4, wobei das Umsetzen mit 3 bis 10 relativen Volumina an Benzonitril, wie 4 bis 6 relativen Volumina an Benzonitril, durchgeführt wird, wobei der Begriff "relatives Volumen" (oder "rel Vol") das Volumen an Lösungsmittel in Litern bedeutet, das relativ zu der Charge der Verbindung von Formel (II), oder einem Salz davon, in Kilogramm erforderlich ist.

6. Verfahren nach einem der Ansprüche 1 bis 5, wobei das Umsetzen mit 1,0 bis 1,5 Moläquivalenten der Verbindung von Formel (III), oder einem Salz davon, durchgeführt wird.

7. Verfahren für die Herstellung einer Verbindung von Formel (IV) 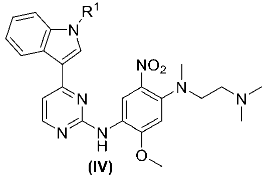
oder eines Salzes davon, umfassend die folgenden Schritte
(i) das Herstellen einer Verbindung von Formel (I), oder eines Salzes davon, nach einem der Ansprüche 1 bis 6; und
(ii) das Herstellen der Verbindung von Formel (IV), oder eines Salzes davon, umfassend die Umsetzung der Verbindung von Formel (I), oder eines Salzes davon, und einer Verbindung von Formel (V) 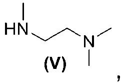
oder eines Salzes davon, in der Gegenwart von Benzonitril,
wobei R¹ C₁₋₃-Alkyl oder Cyclopropyl ist.

8. Verfahren nach Anspruch 7, wobei das Umsetzen der Verbindung von Formel (I), oder eines Salzes davon, und einer Verbindung von Formel (V), oder eines Salzes davon, in der Gegenwart einer Base durchgeführt wird.

9. Verfahren nach Anspruch 8, wobei das Umsetzen der Verbindung von Formel (I), oder eines Salzes davon, und einer Verbindung von Formel (V), oder eines Salzes davon, mit 2,0 bis 2,5 Moläquivalenten der Base durchgeführt wird.

10. Verfahren nach Anspruch 8 oder 9, wobei die Base
(a) 1,8-Diazabicyclo(5.4.0)undec-7-en (DBU), 1,1,3,3-Tetramethylguanidin (TMG), 1,5-Diazabicyclo[4.3.0]non-5-en (DBN), 7-Methyl-1,5,7-triazabicyclo(4.4.0)dec-5-en (MTBD) oder Triazabicyclodecen (TBD), oder
(b) DBU ist.

11. Verfahren nach einem der Ansprüche 7 bis 10, wobei das Umsetzen der Verbindung von Formel (I), oder eines Salzes davon, und einer Verbindung von Formel (V), oder eines Salzes davon, in der Gegenwart eines Fluoridfängers durchgeführt wird.

12. Verfahren nach einem der Ansprüche 7 bis 11, wobei das Umsetzen der Verbindung von Formel (I), oder eines Salzes davon, und einer Verbindung von Formel (V), oder eines Salzes davon, mit 1,8 bis 2,2 Moläquivalenten der Verbindung von Formel (V), oder eines Salzes davon, durchgeführt wird.

13. Verfahren nach einem der Ansprüche 7 bis 12, wobei das Umsetzen der Verbindung von Formel (I), oder eines Salzes davon, und einer Verbindung von Formel (V), oder eines Salzes davon, bei einer Temperatur in dem Bereich von 40 bis 100 °C, wie 70 bis 90 °C, durchgeführt wird.

14. Verfahren nach einem der Ansprüche 7 bis 13, wobei Schritt (i) und Schritt (ii) sequenziell ohne die Isolierung der Verbindung von Formel (I), oder eines Salzes davon, aus dem Benzonitril von Schritt (i) durchgeführt werden.

15. Verfahren nach einem der Ansprüche 1 bis 14, wobei R¹ Methyl ist.

## Revendications

1. Procédé de production d'un composé de formule (I) : 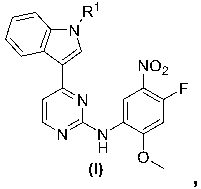
ou un sel de celui-ci, comprenant une réaction d'un composé de formule (II) : 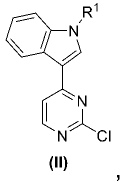
ou un sel de celui-ci, et d'un composé de formule (III) : 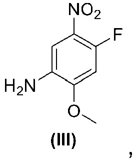
ou un sel de celui-ci, la réaction étant effectuée en présence d'un acide et de benzonitrile,
dans lequel R¹ est alkyle en C₁₋₃ ou cyclopropyle.

2. Procédé selon la revendication 1, dans lequel la réaction est effectuée avec 0,02 à 0,3 équivalent molaire de l'acide, tels que 0,05 à 0,15 équivalent molaire de l'acide.

3. Procédé selon la revendication 1 ou la revendication 2, dans lequel l'acide est
(a) un acide sulfonique,
(b) l'acide méthanesulfonique, l'acide benzènesulfonique ou l'acide p-toluènesulfonique, ou
(c) l'acide méthanesulfonique.

4. Procédé selon l'une quelconque des revendications 1 à 3, dans lequel la réaction est effectuée à une température comprise entre 60 et 130 °C, par exemple entre 90 et 110 °C.

5. Procédé selon l'une quelconque des revendications 1 à 4, dans lequel la réaction est effectuée avec 3 à 10 volumes relatifs de benzonitrile, tel que 4 à 6 volumes relatifs de benzonitrile, le terme « volume relatif » (ou « vol rel ») désignant le volume de solvant en litres nécessaire par rapport à la charge du composé de formule (II), ou d'un sel de celui-ci, en kilogrammes.

6. Procédé selon l'une quelconque des revendications 1 à 5, dans lequel la réaction est effectuée avec 1,0 à 1,5 équivalent molaire du composé de formule (III), ou d'un sel de celui-ci.

7. Procédé de production d'un composé de formule (IV) 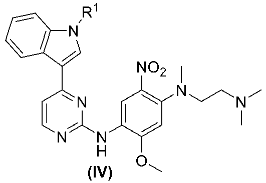
ou d'un sel de celui-ci, comprenant les étapes suivantes
(i) la production d'un composé de formule (I), ou d'un sel de celui-ci, selon l'une quelconque des revendications 1 à 6 ; et
(ii) la production du composé de formule (IV), ou d'un sel de celui-ci, comprenant la réaction du composé de formule (I), ou d'un sel de celui-ci, et d'un composé de formule (V) 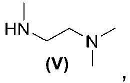
ou d'un sel de celui-ci, en présence de benzonitrile,
dans lequel R¹ est alkyle en C₁₋₃ ou cyclopropyle.

8. Procédé selon la revendication 7, dans lequel la réaction du composé de formule (I), ou d'un sel de celui-ci, et d'un composé de formule (V), ou d'un sel de celui-ci, est effectuée en présence d'une base.

9. Procédé selon la revendication 8, dans lequel la réaction du composé de formule (I), ou d'un sel de celui-ci, et d'un composé de formule (V), ou d'un sel de celui-ci, est effectuée avec 2,0 à 2,5 équivalents molaires de la base.

10. Procédé selon la revendication 8 ou la revendication 9, dans lequel la base est
(a) le 1,8-diazabicyclo(5.4.0)undéc-7-ène (DBU), la 1,1,3,3-tétraméthylguanidine (TMG), le 1,5-diazabicyclo[4.3.0]non-5-ène (DBN), le 7-méthyl-1,5,7-triazabicyclo(4.4.0)déc-5-ène (MTBD) ou le triazabicyclodécène (TBD), ou
(b) le DBU.

11. Procédé selon l'une quelconque des revendications 7 à 10, dans lequel la réaction du composé de formule (I), ou d'un sel de celui-ci, et d'un composé de formule (V), ou d'un sel de celui-ci, est effectuée en présence d'un piégeur de fluorure.

12. Procédé selon l'une quelconque des revendications 7 à 11, dans lequel la réaction du composé de formule (I), ou d'un sel de celui-ci, et d'un composé de formule (V), ou d'un sel de celui-ci, est effectuée avec 1,8 à 2,2 équivalents molaires du composé de formule (V), ou d'un sel de celui-ci.

13. Procédé selon l'une quelconque des revendications 7 à 12, dans lequel la réaction du composé de formule (1), ou d'un sel de celui-ci, et d'un composé de formule (V), ou d'un sel de celui-ci, est effectuée à une température comprise entre 40 et 100 °C, telle qu'entre 70 et 90 °C.

14. Procédé selon l'une quelconque des revendications 7 à 13, dans lequel l'étape (i) et l'étape (ii) sont effectuées séquentiellement sans isolement du composé de formule (I), ou d'un sel de celui-ci, à partir du benzonitrile de l'étape (i).

15. Procédé selon l'une quelconque des revendications 1 à 14, dans lequel R¹ est méthyle.
